# FASCICULE DE BREVET EUROPEEN

(11) **EP 1 409 458 B1**
(45) Date de publication et mention de la délivrance du brevet: **02.08.2006**
(21) Numéro de dépôt: 02787150.8
(22) Date de dépôt: 15.07.2002
(51) Int. Cl.: C07D 211/58, C07D 211/66

(54) **PROCEDE POUR LA PREPARATION DE 4-AMINO-4-PHENYLPIPERIDINES**
VERFAHREN ZUR HERSTELLUNG VON 4 AMINO 4 PHENYLPIPERIDINEN
METHOD FOR PREPARING 4-AMINO-4-PHENYLPIPERIDINES

(30) Priorité: 16.07.2001 FR 0109576
(43) Date de publication de la demande: 21.04.2004
(73) Titulaire: Sanofi-Aventis, 75013 Paris (FR)
(72) Inventeur: ALCADE, Alain, F-31100 Toulouse (FR); ANNE-ARCHARD, Gilles, F-31500 Toulouse (FR); CAPRARO, Daniel, F-31120 Portet sur Garonne (FR)
(74) Mandataire: Varady, Peter
(86) Numéro de dépôt international: PCT/FR2002/002499
(87) Numéro de publication internationale: WO 2003/008382

(56) Documents cités:
- WO-A-01/07050
- US-A- 3 311 624
- IORIO M A ET AL: "NITROGEN ANALOGUES OF PHENCYCLIDINE: 1-ALKYL-4-PHENYL-4-(1-PIPERIDINY L)PIPERIDINES" FARMACO, EDIZIONE SCIENTIFICA, SOCIETA CHIMICA ITALIANA, PAVIA, IT, vol. 39, no. 7, 1984, pages 599-611, XP000872767 ISSN: 0430-0920
- HERMANS BERT ER AL.: "4-Substituted Piperidines. II. Reaction of 1-Benzyl-4-cyano-4-t-aminopiperidines with Organometallic Compounds" J. MED. CHEM., 1965, pages 851-5, XP002198401
- WESTERINGH VAN DE CORNELIS: "4-Substituted Piperidines. I. Deivatives of 4-t-Amino-4-piperidinecarboxamides" J. MED. CHEM., 1964, pages 619-23, XP002198402

## Description

La présente invention concerne un procédé pour la préparation de 4-amino-4-phénylpipéridine et de leurs sels. Ces composés sont utiles comme intermédiaires pour la préparation de principes actifs à usage pharmaceutique, par exemple l'osanétant.

M.A. Iorio et al. (Farmaco, Edizione Scientifica, Societa Chimica Italiana, PAVIA, IT, 1984, 39 n° 7, 599-611) décrivent une synthèse de 1-alkyl-4-phényl-4-(1-pipéridinyl)pipéridine de formule (A) : 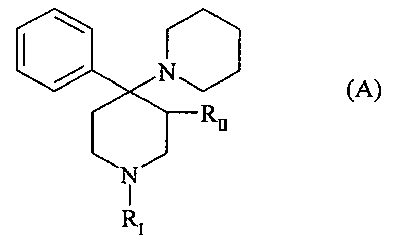 à partir de 4-pipéridone par réaction avec le cyanure de potassium, traitement du 1-alkyl-4-cyanopipéridin-4-ol ainsi obtenu avec la pipéridine et réaction du 1-alkyl-4-cyano-4-(1-pipéridinyl)pipéridine ainsi obtenu avec le bromure de phénylmagnésium. Toutefois il est décrit dans ce document que l'hydrogénolyse d'un composé de formule (A) dans laquelle R₁ = benzyle suivi d'une hydrogénation conduit uniquement à l'obtention de 4-phénylpipéridine par élimination de la pipéridine en C-4, et ne conduit pas à la préparation d'un composé de formule (A) dans laquelle R₁ = H.

G.A.M. Giardina *et al.* (Bioorg. Med. and Chem. Letters, 1996, 6, 2307-2310) décrivent une synthèse de la 4-méthylamino-4-phénylpipéridine de formule (C): 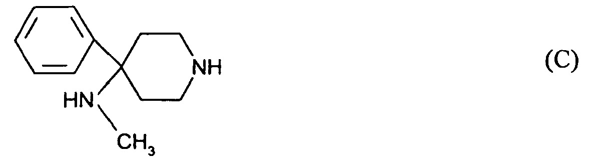 en six étapes à partir de la 1-benzyl-4-pipéridone par réaction avec le phényllithium, traitement du 1-benzyl-4-phénylpipéridin-4-ol ainsi obtenu avec l'acide sulfurique et l'acide acétique dans l'acétonitrile, désacétylation de la 4-acétamido-1-benzyl-4-phénylpipéridine, N-formylation de la 4-amino-1-benzyl-4-phénylpipéridine, réduction du dérivé N-formylé ainsi obtenu avec de l'hydrure de lithium et d'aluminium et débenzylation finale de la 1-benzyl-4-méthylamino-4-phénylpipéridine par hydrogénation catalytique.

Bien que ces six étapes se déroulent avec de bons rendements, le procédé présente des inconvénients qui en rendent peu aisée l'application industrielle. Plus particulièrement, dans la préparation du composé (C), deux des six étapes sont conduites à reflux pendant trois jours, ce qui implique une durée du procédé considérable. De plus, la préparation du composé (C) comporte, dans la première étape, l'utilisation du phényllithium qui peut entraîner des problèmes non négligeables au niveau industriel.

Il a été maintenant trouvé qu'à partir de la 1-benzyl-4-pipéridone ou, de façon plus générale, de la 4-pipéridone 1-protégée, il est possible de préparer la 4-méthylamino-4-phénylpipéridine (composé C ci-dessus) en quatre étapes seulement, dont les deux premières peuvent éventuellement se dérouler l'une après l'autre dans le même réacteur (réaction "one pot"), à partir de la cyanhydrine, une réaction avec la benzylméthylamine (ou, de façon plus générale la méthylamine N-protégée), une réaction avec le bromure de phénylmagnésium et une déprotection des atomes d'azote de la pipéridine et de la méthylamine en position 4 de la pipéridine.

Il a été également trouvé qu'en remplaçant la méthylamine N-protégée par une autre alkylamine N-protégée, on peut préparer d'autres 4-alkylamino-4-phénylpipéridines dont la synthèse n'est pas possible en utilisant la méthode proposée par G.A.M. Giardina *et al.* dans le document cité ci-dessus.

Il a été notamment constaté qu'en opérant via la cyanhydrine et son produit de réaction avec l'amine, puis par réaction de Grignard, on peut choisir les conditions réactionnelles ainsi que les groupes protecteurs des deux atomes d'azote de façon à passer par des intermédiaires convenables. Par conséquent le procédé *via* la cyanhydrine et le réactif de Grignard permet de préparer des 4-amino-4-phénylpipéridines différemment substituées.

Plus particulièrement, il a été trouvé qu'en remplaçant la benzylméthylamine (ou, de façon plus générale, la méthylamine N-protégée) par la dibenzylamine (ou, de façon plus générale, l'ammoniac substitué par deux groupes N-protecteurs), on obtient de façon très simple la 4-amino-4-phénylpipéridine, éventuellement 4-N-protégée.

Ainsi, selon un des ses aspects, la présente invention a pour objet un procédé pour la préparation d'une 4-amino-4-phénylpipéridine de formule (I) 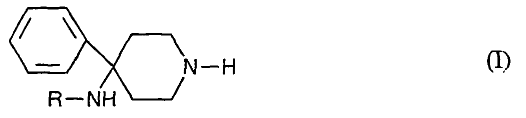 dans laquelle R est l'hydrogène ou un groupe (C₁-C₃)alkyle, caractérisé en ce que :
(a) on traite de façon séquentielle une 4-pipéridone 1-protégée de formule 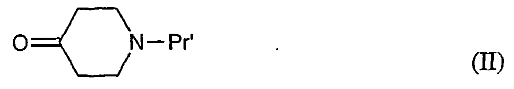 dans laquelle Pr' représente un groupe N-protecteur éliminable, d'abord avec un cyanure alcalin et ensuite, dans le mélange réactionnel contenant la cyanhydrine ainsi obtenue, avec une amine de formule : 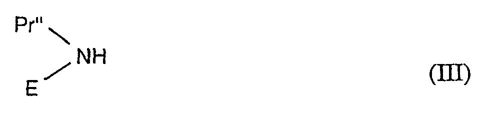 dans laquelle E représente un groupe R' = (C₁-C₃)alkyle, ou un groupe N-protecteur, le ou les groupes protecteurs étant éliminables dans les mêmes conditions que Pr' ;
(b) on soumet le composé ainsi obtenu de formule : 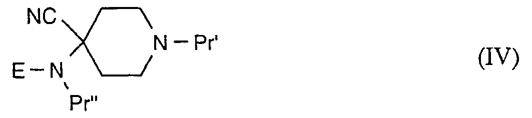 dans laquelle Pr', Pr" et E sont tels que définis ci-dessus, à une réaction de Grignard avec un halogénure de phénylmagnésium ; et
(c) on élimine les deux ou trois groupes N-protecteurs du composé ainsi obtenu de formule : 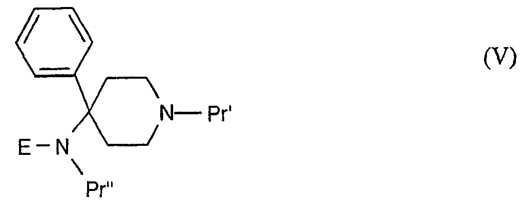 et on isole le composé (I) soit sous forme d'un de ses sels que l'on transforme en la base libre, soit sous forme de base libre que l'on transforme en l'un des ses sels.

Dans l'étape (a), on opère dans les conditions habituelles de formation des cyanhydrines, en utilisant avantageusement du cyanure de potassium en tant que cyanure alcalin et un composé de formule (II) dans laquelle Pr' est un radical benzyle, non substitué ou substitué sur le cycle benzénique par un halogène, un groupe (C₁-C₄)alkyle ou un groupe (C₁-C₄)alcoxy ; un radical benzhydryle ; ou un radical trityle, de préférence un radical benzyle.

Après élimination de la phase aqueuse, le mélange réactionnel, dans un solvant aqueux-organique, de préférence dans un mélange eau/toluène ou eau/éthanol, est directement traité avec le composé de formule (III). Avantageusement, on utilise un composé de formule (III) dans laquelle Pr" est un radical benzyle, non substitué ou substitué sur le cycle benzénique par un halogène, un groupe (C₁-C₄)alkyle, un groupe (C₁-C₄)alcoxy ; un radical benzhydryle ou un radical trityle, de préférence un radical benzyle.

Dans le composé (III), E est avantageusement (C₁-C₃)alkyle, de préférence méthyle.

Dans le composé (III), E peut également être un groupe N-protecteur tel qu'un radical benzyle, non substitué ou substitué sur le cycle benzénique par un halogène, un (C₁-C₄)alkyle ou un (C₁-C₄)alcoxy ; un radical benzhydryle ou un radical trityle, de préférence un radical benzyle.

On opère ensuite dans le solvant, avantageusement dans le toluène en présence de sulfate de magnésium ou dans un mélange eau/éthanol. Dans ces conditions, après 2 à 12 heures à une température de 30 à 45°C, la réaction est complète et le composé de formule (IV) est isolé selon les techniques conventionnelles :
- par exemple en ajoutant de l'eau et en éliminant les sous-produits par des traitements avec un acide, par exemple l'acide acétique, et une base, par exemple l'hydroxyde de sodium, en éliminant la phase aqueuse et en évaporant le solvant organique, avantageusement le toluène :
- ou par cristallisation à partir du mélange éthanol/eau.

Dans l'étape (b), l'halogénure de phénylmagnésium est préparé extemporanément à partir d'un halogénobenzène et de magnésium. De préférence, on prépare le bromure de phénylmagnésium à partir de bromobenzène et de magnésium dans un mélange toluène/*tert*-butyléther de méthyle ou toluène/tétrahydrofurane. Une solution du composé de formule (IV) dans le toluène, est ajoutée à la solution contenant l'halogénure de phénylmagnésium et, après 1-2 heures à la température ambiante, le complexe ainsi formé est hydrolysé et le composé (V) est isolé selon les méthodes conventionnelles, par exemple comme illustré ci-dessus pour l'isolement du composé de formule (IV). Le composé (V) est isolé sous forme de base libre ou d'un de ses sels.

Dans l'étape (c) les groupes protecteurs Pr' et Pr" et éventuellement E sont éliminés en même temps. Lorsque, dans le composé de formule (V) ainsi obtenu, Pr' et Pr" et éventuellement E sont un radical benzyle, non substitué ou substitué sur le cycle benzénique par un halogène, un groupe (C₁-C₄)alkyle ou un groupe (C₁-C₄)alcoxy ; un radical benzhydryle ; ou un radical trityle, de préférence un radical benzyle, la déprotection est aisément effectuée par hydrogénation catalytique dudit composé (V), sous forme de base libre ou d'un de ses sels. Avantageusement, on utilise le palladium sur charbon (Pd/C) en tant que catalyseur.

Le composé de formule (I) est isolé sous forme de base libre lorsque le composé de départ (V) était sous forme de base libre ou bien sous forme du même sel que celui du composé (V) salifié, lorsque le composé (V) soumis à l'hydrogénation était sous forme de sel. Dans ce dernier cas, la base libre peut être obtenue par neutralisation.

A l'étape a), on utilise une amine de formule (III) dans laquelle E représente un groupe N-protecteur pour préparer un composé de formule (I) dans laquelle R est l'hydrogène. Préférentiellement, on utilise à l'étape a) une amine de formule (III) dans laquelle E représente un groupe R' = (C₁-C₃)alkyle.

Plus particulièrement, la présente invention concerne également un procédé pour la préparation des composés de formule (I), dans laquelle R est autre que l'hydrogène.

Les composés de formule (IV) et de formule (V), dans lesquelles R' est tel que défini ci-dessus et Pr' et Pr" sont les radicaux avantageux cités ci-dessus et leurs sels sont des composés nouveaux qui constituent les intermédiaires-clés du procédé de la présente invention.

Ainsi, selon un autre de ses aspects, la présente invention a pour objet les composés de formule : 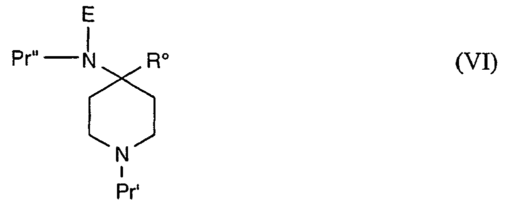 dans laquelle E est un (C₁-C₃)alkyle, un radical benzyle, non substitué ou substitué sur le cycle benzénique par un halogène, un (C₁-C₄)alkyle ou un (C₁₋C₄)alcoxy ; un radical benzhydryle ou un radical trityle, R° est un groupe cyano ou phényle, Pr' et Pr", indépendamment l'un de l'autre, représentent un radical benzyle, non substitué ou substitué sur le cycle benzénique par un halogène, un groupe (C₁-C₄)alkyle ou (C₁-C₄)alcoxy ; un radical benzhydryle ; un radical trityle ; et leurs sels.

Parmi les composés de formule (VI), on préfère ceux pour lesquels E et un (C₁-C₃)alkyle ; les composés pour lesquels E est méthyle, étant particulièrement avantageux.

Les composés de formule (VI) dans laquelle Pr' et Pr" sont tous les deux benzyle sont également très avantageux et, parmi ces composés,
- la 1-benzyl-4-[(N-benzyl)méthylamino)]-4-cyanopipéridine et ses sels, ainsi que
- la 1-benzyl-4-[(N-benzyl)méthylamino]-4-phénylpipéridine et ses sels, notamment le dioxalate, sont préférés.

### EXEMPLE 1

(a) A un mélange de 83,8 g de cyanure de potassium, 100 ml d'eau et 750 ml de toluène, refroidi à 15°C, on ajoute sous agitation, en 3 heures, une solution aqueuse de chlorhydrate de 1-benzyl-4-pipéridone (préparée à partir de 221,6 g de 1-benzyl-4-pipéridone, 60 g de glace, 60 ml d'eau et 100 ml d'HCl à 36 %). Le mélange ainsi obtenu est chauffé à 45 °C, tout en capturant les gaz éventuellement dégagés par neutralisation, puis on élimine la phase aqueuse après décantation, on refroidit la phase toluénique à 20 °C, on y ajoute 279 g de sulfate de magnésium et, à 45 °C, 160 g de N-benzylméthylamine. Après 6 heures à cette température on ajoute progressivement, à 40°C, 750 ml d'eau. Toujours à cette température, on laisse décanter, on élimine la phase aqueuse, puis on refroidit à 15 °C, on ajoute 500 ml d'eau et 20.g d'acide acétique glacial. On laisse décanter, on élimine la phase aqueuse, on lave la phase organique d'abord à l'eau, puis avec de l'hydroxyde de sodium dilué, et enfin, encore avec de l'eau, on sèche la phase toluénique par azéotropie, on évapore à sec sous vide et on sèche le résidu solide sous vide à 40°C. On obtient ainsi 345,5 g de 1-benzyl-4-cyano-4-[(N-méthyl)benzylamino]pipéridine ; F = 67-68 °C.
(b) A 2,11 1 d'une solution de bromure de phénylmagnésium dans un mélange toluène/*tert*-butyléther de méthyle, obtenue à partir de 486,6 g de bromobenzène et 75,32 g de magnésium et refroidie à 15 °C, on ajoute 330 g du produit obtenu à la fin de l'étape (a). On laisse le mélange réactionnel pendant deux heures à la température ambiante, puis on le traite avec 1,5 1 d'eau glacée, 100 ml d'eau oxygénée à 30 % en 90 minutes à 2°C et on le laisse sous agitation pendant 30 minutes. On décante et on élimine la phase aqueuse, on lave la phase organique à l'eau. On traite par une solution de 70 ml d'acide acétique dans 765 ml d'eau pour atteindre un pH entre 6,5 et 7,5. On traite la phase organique avec de l'acide chlorhydrique dilué, on l'élimine et on traite la phase aqueuse avec 1,5 1 de *n*-butanol. On ajoute de l'hydroxyde de sodium jusqu'à pH 14, on décante et on lave la phase organique à chaud (60 °C) avec de l'eau. On élimine la phase aqueuse et on sèche la phase organique par azéotropie. On refroidit à 70 °C, on ajoute 2,24 1 d'éthanol absolu puis une solution de 185 g d'acide oxalique dans 180 ml d'éthanol et on chauffe le mélange à reflux pendant 1 heure. On laisse revenir à 20°C, on filtre le précipité et on lave à l'éthanol. On obtient ainsi 382 g de dioxalate de 1-benzyl-4-(N-méthyl)benzylamino-4-phénylpipéridine ; F = 179-180°C.
(c) Un mélange de 300 g du produit obtenu à la fin de l'étape (b), 1,51 de méthanol et 30 g de Pd/C à 5 % contenant 50 % d'humidité est hydrogéné à 45 °C et à la pression ambiante pendant 24 heures, puis on y ajoute 300 ml d'eau et on le chauffe pendant 30 minutes à reflux. A cette température le mélange est filtré, le méthanol est éliminé par distillation et la phase essentiellement aqueuse est chauffée à 95°C. On ajoute 1,7 1 de *n*-butanol tout en maintenant la température à 95°C, puis on chauffe à reflux, on refroidit à 20°C, on filtre le précipité, on le lave avec un mélange butanol/eau 9/1 (v/v) et on le sèche à 60 °C sous vide. On obtient ainsi 144,25 g de sesquioxalate de 4-méthylamino-4-phénylpipéridine monohydraté ; F = 252-254°C (capillaire).

### EXEMPLE 2

(a) A un mélange de 41,9 g de cyanure de potassium, 50 ml d'eau et 380 ml de toluène, refroidi à 15 °C, on ajoute sous agitation, en 3 heures, une solution aqueuse de chlorhydrate de 1-benzylpipéridin-4-one (préparée à partir de 110,8 g de 1-benzyl-4-pipéridone, 30 ml d'eau, 30 g de glace et 50 ml d'HCl à 36 %). Le mélange ainsi obtenu est chauffé à 45 °C tout en capturant les gaz éventuels dégagés par neutralisation, puis on écarte la phase aqueuse, on refroidit la phase toluénique à 20 °C, on y ajoute 140 g de sulfate de magnésium et à 45°C, 90,7 g de N-benzyléthylamine. Après 10 heures à cette température, on ajoute progressivement, à 40 °C, 380 ml d'eau, on laisse décanter à la même température, on élimine la phase aqueuse, puis on refroidit à 15 °C, on ajoute 250 ml d'eau et 10 g d'acide acétique glacial. On élimine la phase aqueuse, on lave la phase organique à l'eau puis avec de l'hydroxyde de sodium dilué et enfin encore à l'eau. On sèche la phase toluénique par azéotropie, on évapore à sec sous vide et on sèche le résidu solide à 40 °C sous vide. On obtient ainsi 193,2 g de 1-benzyl-4-cyano-4-[(N-éthyl)benzylamino] pipéridine.
(b) A 1,05 1 d'une solution de bromure de phénylmagnésium dans un mélange de toluène/*tert*-butyléther de méthyle, obtenue de 243,3 g de bromobenzène et 37,66 g de magnésium et refroidie à 15 °C, on ajoute 193 g du produit obtenu à la fin de l'étape (a). On laisse le mélange pendant 2 heures à la température ambiante, puis on le traite en 75 minutes et à -2 °C, avec 750 ml d'eau glacée, 50 ml d'eau oxygénée à 30 %. On laisse le mélange ainsi obtenu sous agitation pendant 30 minutes. On traite par une solution de 35 ml d'acide acétique dans 380 ml d'eau pour atteindre un pH compris entre 6,5 et 7,5. On élimine la phase aqueuse et on lave la phase organique à l'eau. On traite la phase organique avec de l'acide chlorhydrique dilué et on l'élimine. La phase aqueuse est traitée avec 750 ml de *n*-butanol, puis avec une solution d'hydroxyde de sodium jusqu'à pH 14. On décante la phase aqueuse, on lave la phase organique à l'eau à 60 °C, puis on élimine la phase aqueuse et on sèche la solution butanolique par azéotropie. On ajoute 1,1 1 d'éthanol absolu à 65-70 °C, puis à la même température, une solution de 92,5 g d'acide oxalique dans 90 ml d'éthanol. On chauffe le mélange à reflux pendant 30 minutes, puis on refroidit, on filtre le précipité et on le lave avec de l'éthanol froid. On obtient ainsi 196,5 g de dioxalate de 1-benzyl-4-(N-éthyl)benzylamino-4-phénylpipéridine.
(c) Un mélange de 150 g du produit obtenu à la fin de l'étape (b), 750 ml de méthanol et 15 g de Pd/C à 5 % contenant 50 % d'humidité est hydrogéné à 45 °C et à la pression ambiante pendant 24 heures, puis on y ajoute 150 ml d'eau et on le chauffe 30 minutes à reflux. A cette température, le mélange est filtré, le méthanol et l'eau sont éliminés par distillation. Le résidu solide est repris avec 2,5 1 d'acétone et l'on ajuste le pH à 2,5-3 avec de l'acide oxalique. On agite pendant 2 heures à température ambiante, on filtre le précipité on le lave avec de l'acétone et on le sèche à 60°C sous vide. On obteint ainsi 85,7 g de dioxalate de 4-éthylamino-4-phénylpipéridine.

### EXEMPLE 3

(a) A un mélange de 83,8 g de cyanure de potassium, 100 ml d'eau et 750 ml de toluène, refroidi à 15°C, on ajoute sous agitation, en 3 heures, une solution aqueuse de chlorhydrate de 1-benzyl-4-pipéridone (préparée à partir de 221,6 g de 1-benzyl-4-pipéridone, 60 g de glace, 60 ml d'eau et 100 ml d'HCl à 36 %). Le mélange ainsi obtenu est chauffé à 45°C, tout en capturant les gaz éventuellement dégagés par neutralisation, puis on élimine la phase aqueuse, après décantation, on refroidit la phase toluénique à 20°C, on y ajoute 279 g de sulfate de magnésium et, à 45°C, 160 g de N-benzylméthylamine. Après 6 heures à cette température on ajoute progressivement, à 40°C, 750 ml d'eau. Toujours à cette température, on laisse décanter, on élimine la phase aqueuse, puis on refroidit à 15°C, on ajoute 500 ml d'eau et 20 g d'acide acétique glacial. On laisse décanter, on élimine la phase aqueuse, on lave la phase organique d'abord à l'eau, puis avec de l'hydroxyde de sodium dilué, et enfin, encore avec de l'eau, on sèche la phase toluénique par azéotropie, on évapore à sec sous vide et on sèche le résidu solide sous vide à 40°C. On obtient ainsi 345,5 g de 1-benzyl-4-cyano-4-[(N-méthyl)benzylamino]pipéridine ; F = 67-68 °C.
(b) A 2,11 1 d'une solution de bromure de phénylmagnésium dans un mélange toluène/*tert*-butyléther de méthyle, obtenue à partir de 486,6 g de bromobenzène et 75,32 g de magnésium et refroidie à 15 °C, on ajoute 330 g du produit obtenu à la fin de l'étape (a). On laisse le mélange réactionnel pendant deux heures à la température ambiante, puis on le traite avec 1,51 d'eau glacée, 100 ml d'eau oxygénée à 30 % en 90 minutes à 2°C et on le laisse sous agitation pendant 30 minutes. On décante et élimine la phase aqueuse, on lave la phase organique à l'eau. On la traite par une solution de 70 ml d'acide acétique dans 765 ml d'eau pour atteindre un pH entre 6,5 et 7,5. On traite la phase organique avec de l'acide chlorhydrique dilué, on élimine et on traite la phase aqueuse avec 1,5 1 de *n*-butanol. On ajoute de l'hydroxyde de sodium jusqu'à pH 14, on décante et on lave la phase organique à chaud (60 °C) avec de l'eau. On élimine la phase aqueuse et on sèche la phase organique par azéotropie. On refroidit à 70 °C, on ajoute 2,24 1 d'éthanol absolu puis une solution de 185 g d'acide oxalique dans 180 ml d'éthanol et on chauffe le mélange à reflux pendant 1 heure. On laisse revenir à 20°C, on filtre le précipité et on lave à l'éthanol. On obtient ainsi 382 g de dioxalate de 1-benzyl-4-(N-méthyl)benzylamino-4-phénylpipéridine ; F = 179-180°C.
(c) Un mélange de 300 g du produit obtenu à la fin de l'étape (b), 1,5 1 de méthanol et 30 g de Pd/C à 5 % contenant 50 % d'humidité est hydrogéné à 45 °C et à la pression ambiante pendant 24 heures, puis on y ajoute 300 ml d'eau et on le chauffe pendant 30 minutes à reflux. A cette température le mélange est filtré, le méthanol est éliminé par distillation et la phase essentiellement aqueuse est chauffée à 95°C. On ajoute 1,7 1 de *n*-butanol tout en maintenant la température à 95 °C, puis on chauffe à reflux, on refroidit à 20°C, on filtre le précipité, on le lave avec un mélange butanol/eau 9/1 (v/v) et on le sèche à 60°C sous vide. On obtient ainsi 144,25 g de sesquioxalate de 4-méthylamino-4-phénylpipéridine monohydraté ; F = 252-254°C (capillaire).

## Revendications

1. Procédé pour la préparation d'une 4-amino-4-phénylpipéridine de formule (I) : 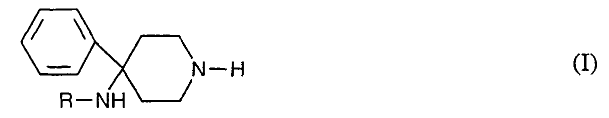 dans laquelle R est l'hydrogène ou un groupe (C₁-C₃)alkyle, **caractérisé en ce que** :
(a) on traite de façon séquentielle une 4-pipéridone 1-protégée de formule : 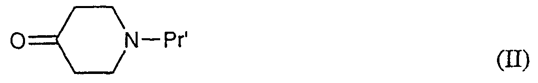 dans laquelle Pr' représente un groupe N-protecteur éliminable, d'abord avec un cyanure alcalin et ensuite, dans le mélange réactionnel contenant la cyanhydrine ainsi obtenue, avec une amine de formule : 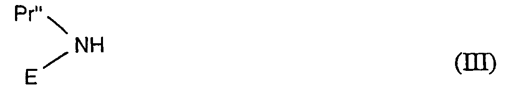 dans laquelle E représente un groupe R' = (C₁-C₃)alkyle ou un groupe N-protecteur et Pr" est un groupe N-protecteur, le ou les groupes protecteurs étant éliminables dans les mêmes conditions que Pr' ;
(b) on soumet le composé ainsi obtenu de formule : 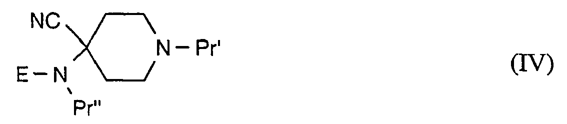 dans laquelle Pr', Pr" et E sont tels que définis ci-dessus, à une réaction de Grignard avec un halogénure de phénylmagnésium ; et
(c) on élimine les deux ou trois groupes protecteurs du composé ainsi obtenu de formule : 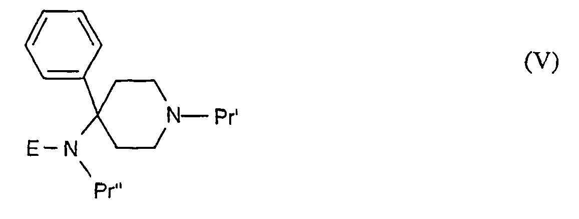 et on isole le composé (I) soit sous forme d'un de ses sels que l'on transforme en la base libre, soit sous forme de base libre que l'on transforme en l'un de ses sels.

2. Procédé selon la revendication 1 pour la préparation d'une 4-amino-4-phényl-pipéridine de formule : 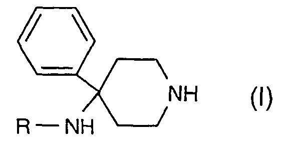 dans laquelle R représente un groupe (C₁-C₃)alkyle, **caractérisé en ce que**, à l'étape a), on traite par une amine de formule Pr"ENH dans laquelle E est un groupe (C₁-C₃)alkyle, Pr" est tel que défini dans la revendication 1.

3. Procédé selon l'une des revendications 1 ou 2, **caractérisé en ce que**, comme réactifs de départ, on utilise un composé de formule (II) et un composé de formule (III) dans lesquelles E représente un groupe R' = (C₁₋C₃) alkyle et Pr' et Pr" représentent, indépendamment, un radical benzyle, non substitué ou substitué sur le cycle benzénique par un halogène, un groupe (C₁-C₄)alkyle ou un groupe (C₁-C₄)alcoxy ; un radical benzhydryle ; ou un radical trityle.

4. Procédé selon l'une quelconque des revendications 1 à 3, **caractérisé en ce que** comme réactif de départ, on utilise un composé de formule (II), dans laquelle Pr' est un groupe benzyle, et un composé de formule (III), dans laquelle Pr" est également un groupe benzyle.

5. Procédé selon l'une quelconque des revendications 1 à 4, **caractérisé en ce que** le composé de formule (IV) dans laquelle Pr' et Pr" sont tous les deux un groupe benzyle, est soumis, dans l'étape b) à une réaction de Grignard avec du bromure de phénylmagnésium.

6. Procédé selon l'une quelconque des revendication 1 à 5, **caractérisé en ce que** comme réactif de départ on utilise un composé de formule (III) dans E est un groupe méthyle.

7. Composé de formule : 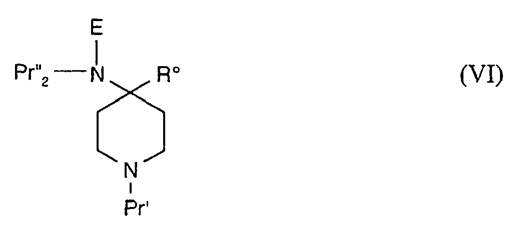 dans laquelle E est un groupe (C₁-C₃)alkyle, un radical benzyle, non substitué ou substitué sur le cycle benzénique par un halogène, un (C₁₋C₄)alkyle ou (C₁-C₄)alcoxy ; un radical benzhydryle ou un radical trityle, R° est un groupe cyano ou phényle, Pr' et Pr", indépendamment l'un de l'autre, représentent un radical benzyle, non substitué ou substitué sur le cycle benzénique par une halogène, un groupe (C₁-C₄)alkyle ou (C₁₋C₄)alcoxy ; un radical benzhydryle , un radical trityle, et ses sels.

8. Composé de formule (VI) selon la revendication 7 dans laquelle E est un groupe (C₁-C₃)alkyle, R°, Pr' et Pr" étant tels que définis dans la revendication 7 et leurs sels.

9. 1-Benzyl-4-[(N-benzyl)méthylamino)]1-4-cyanopipéridine et ses sels.

10. 1-Benzyl-4-[(N-benzyl)méthylamino]-4-phénylpipéridine et ses sels.

11. Dioxalate de 1-benzyl-4-[(N-benzyl)méthylamino]-4-phénylpipéridine.

12. Sesquioxalate de 1-benzyl-4-[(N-benzyl)méthylamino]-4-phénylpipéridine.

## Claims

1. A process for preparing a 4-amino-4-phenylpiperidine of formula (I): 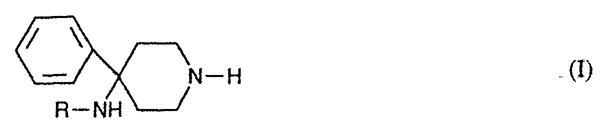 in which R is hydrogen or a (C₁-C₃)alkyl group,
**characterized in that**:
(a) a 1-protected 4-piperidone of formula 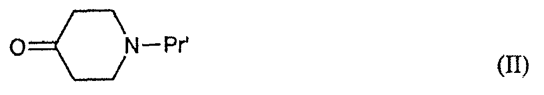 in which Pr' represents a removable N-protecting group, is treated sequentially first with an alkali metal cyanide and then, in the reaction medium containing the cyanohydrin thus obtained, with an amine of formula: 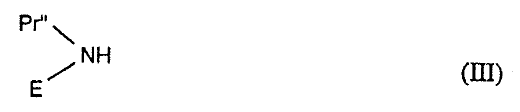 in which E represents a group R' = (C₁-C₃)alkyl, or an N-protecting group and Pr" is an N-protecting group, the protecting group(s) being removable under the same conditions as Pr';
(b) the compound thus obtained of formula: 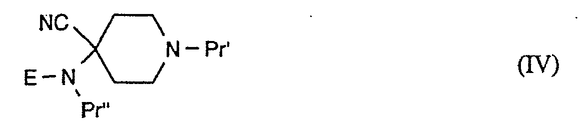 in which Pr', Pr" and E are as defined above, is subjected to a Grignard reaction with a phenylmagnesium halide; and
(c) the two or three protecting groups are removed from the compound thus obtained of formula: 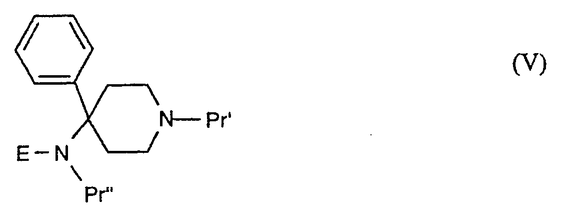 and compound (I) is isolated either in the form of one of its salts, which is converted into the free base, or in the form of the free base, which is converted into one of its salts.

2. Process according to Claim 1 for the preparation of a 4-amino-4-phenylpiperidine of formula: 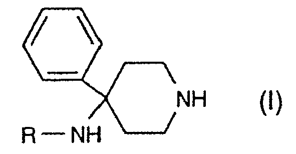 in which R represents a (C₁-C₃)alkyl group, **characterized in that**, in step a), treatment is performed with an amine of formula Pr"ENH in which E is a (C₁-C₃)alkyl group, and Pr" is as defined in Claim 1.

3. Process according to either of Claims 1 and 2, **characterized in that** the starting reagents used are a compound of formula (II) and a compound of formula (III) in which E represents a group R' = (C₁₋C₃)alkyl and Pr' and Pr" represent, independently, a benzyl radical, which is unsubstituted or substituted on the benzene ring with a halogen, a (C₁-C₄) alkyl group or a (C₁-C₄)alkoxy group; a benzhydryl radical; or a trityl radical.

4. Process according to any one of Claims 1 to 3, **characterized in that** the starting reagents used are a compound of formula (II) in which Pr' is a benzyl group, and a compound of formula (III) in which Pr" is also a benzyl group.

5. Process according to any one of Claims 1 to 4, **characterized in that** the compound of formula (IV) in which Pr' and Pr" are both a benzyl group is subjected, in step b), to a Grignard reaction with phenylmagnesium bromide.

6. Process according to any one of Claims 1 to 5, **characterized in that** the starting reagent used is a compound of formula (III) in which E is a methyl group.

7. Compound of formula: 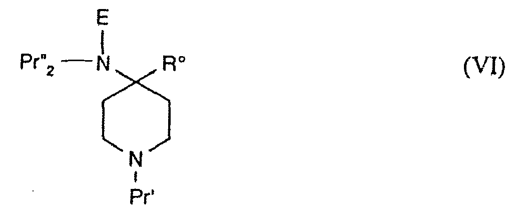 in which E is a (C₁-C₃)alkyl group, a benzyl radical, which is unsubstituted or substituted on the benzene ring with a halogen, a (C₁-C₄) alkyl or a (C₁-C₄) alkoxy; a benzhydryl radical or a trityl radical, R° is a cyano or phenyl group, Pr' and Pr", independently of each other, represent a benzyl radical, which is unsubstituted or substituted on the benzene ring with a halogen, a (C₁-C₄) alkyl or (C₁-C₄) alkoxy group; a benzhydryl radical, a trityl radical, and its salts.

8. Compound of formula (VI) according to Claim 7, in which E is a (C₁-C₃)alkyl group, R°, Pr' and Pr" being as defined in Claim 7, and salts thereof.

9. 1-Benzyl-4-[(N-benzyl)methylamino)]-4-cyanopiperidine and its salts.

10. 1-Benzyl-4-[(N-benzyl)methylamino]-4-phenylpiperidine and its salts.

11. 1-Benzyl-4-[(N-benzyl)methylamino]-4-phenylpiperidine dioxalate.

12. 1-Benzyl-4-[(N-benzyl)methylamino]-4-phenylpiperidine sesquioxalate.

## Patentansprüche

1. Verfahren zur Herstellung eines 4-Amino-4-phenyl-piperidins der Formel (I): 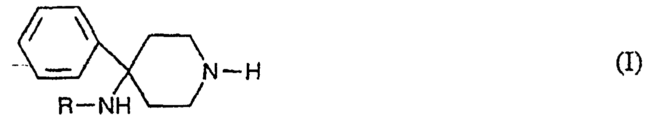 worin R für Wasserstoff oder eine (C₁-C₃) -Alkylgruppe steht, **dadurch gekennzeichnet, daß** man:
(a) ein 1-geschütztes 4-Piperidon der Formel: 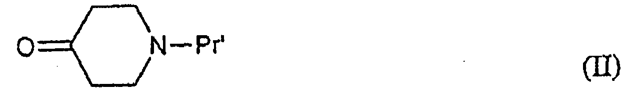 worin Pr' für eine abspaltbare N-Schutzgruppe steht, nacheinander zunächst mit einem Alkalimetallcyanid und dann in der das so erhaltene Cyanhydrin enthaltenden Reaktionsmischung mit einem Amin der Formel: 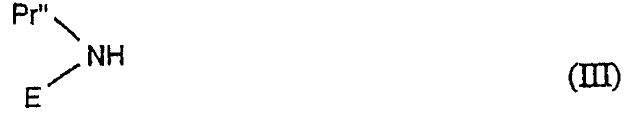 worin E für eine Gruppe R' = (C₁-C₃)-Alkyl oder eine N-Schutzgruppe steht und Pr" für eine N-Schutzgruppe steht, wobei die Schutzgruppe bzw. die Schutzgruppen unter den gleichen Bedingungen abspaltbar sind wie Pr', behandelt;
(b) die so erhaltene Verbindung der Formel: 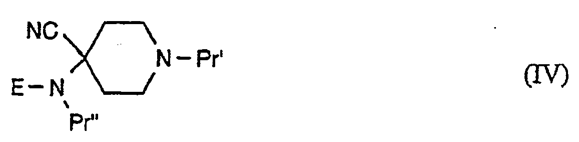 worin Pr', Pr" und E die oben angegebene Bedeutung besitzen, einer Grignard-Reaktion mit einem Phenylmagnesiumhalogenid unterwirft und
(c) die zwei oder drei Schutzgruppen der so erhaltenen Verbindung der Formel: 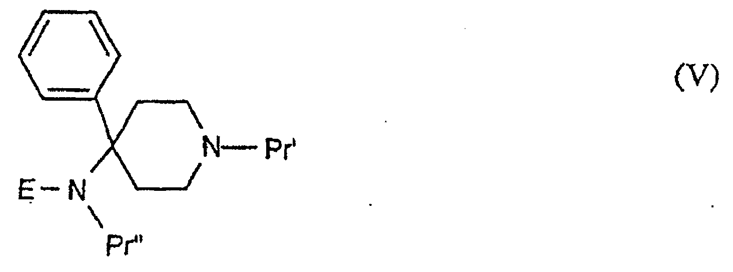 abspaltet und die Verbindung (I) entweder in Form eines Salzes davon, welches man in die freie Base umwandelt, oder in Form der freien Base, welche man in ein Salz davon umwandelt, isoliert.

2. Verfahren nach Anspruch 1 zur Herstellung eines 4-Amino-4-phenylpiperidins der Formel (I): 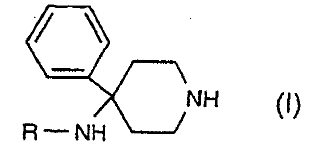 worin R für eine (C₁-C₃)-Alkylgruppe steht, **dadurch gekennzeichnet, daß** man in Schritt a) die Behandlung mit einem Amin der Formel Pr" ENH, worin E für eine (C₁-C₃)-Alkylgruppe steht und Pr" die in Anspruch 1 angegebene Bedeutung besitzt, durchführt.

3. Verfahren nach Anspruch 1 oder 2, **dadurch gekennzeichnet, daß** man als Ausgangsstoffe eine Verbindung der Formel (II) und eine Verbindung der Formel (III), worin E für eine Gruppe R' = (C₁-C₃)-Alkyl steht und Pr' und Pr" unabhängig voneinander für einen Benzylrest, der gegebenenfalls am Benzolring durch ein Halogen, eine (C₁-C₄)-Alkylgruppe oder eine (C₁-C₄)-Alkoxygruppe substituiert ist, einen Benzhydrylrest oder einen Tritylrest stehen, verwendet.

4. Verfahren nach einem der Ansprüche 1 bis 3,
**dadurch gekennzeichnet, daß** man als Ausgangsstoff eine Verbindung der Formel (II), worin Pr' für eine Benzylgruppe steht, und eine Verbindung der Formel (III), worin Pr" ebenfalls für eine Benzylgruppe steht, verwendet.

5. Verfahren nach einem der Ansprüche 1 bis 4,
**dadurch gekennzeichnet, daß** man die Verbindung der Formel (IV), worin Pr' und Pr" beide für eine Benzylgruppe stehen, in Schritt b) einer Grignard-Reaktion mit Phenylmagnesiumbromid unterwirft.

6. Verfahren nach einem der Ansprüche 1 bis 5,
**dadurch gekennzeichnet, daß** man als Ausgangsstoff eine Verbindung der Formel (III), worin E für eine Methylgruppe steht, verwendet.

7. Verbindung der Formel: 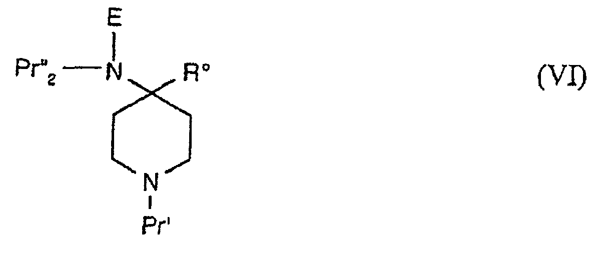 worin E für eine (C₁-C₃)-Alkylgruppe, einen Benzylrest, der gegebenenfalls am Benzolring durch ein Halogen, eine (C₁-C₄)-Alkylgruppe oder eine (C₁-C₄)-Alkoxygruppe substituiert ist, einen Benzhydrylrest oder einen Tritylrest steht, R° für eine Cyano- oder Phenylgruppe steht und Pr' und Pr" unabhängig voneinander für einen Benzylrest, der gegebenenfalls am Benzolring durch ein Halogen, eine (C₁-C₄) -Alkylgruppe oder eine (C₁-C₄) -Alkoxygruppe substituiert ist, einen Benzhydrylrest oder einen Tritylrest stehen, und Salze davon.

8. Verbindung der Formel (VI) nach Anspruch 7, worin E für eine (C₁-C₃) -Alkylgruppe steht und R°, Pr' und Pr" die in Anspruch 7 angegebene Bedeutung besitzen, und Salze davon.

9. 1-Benzyl-4-[(N-benzyl)methylamino)]-4-cyano-piperidin und Salze davon.

10. 1-Benzyl-4-[(N-benzyl)methylamino)]-4-phenyl-piperidin und Salze davon.

11. 1-Benzyl-4-[(N-benzyl)methylamino)]-4-phenyl-piperidin-dioxalat.

12. 1-Benzyl-4-[(N-benzyl)methylamino)]-4-phenyl-piperidin-sesquioxalat.
